(19) Europäisches Patentamt
European Patent Office
Office européen des brevets

(11) **EP 2 033 579 A1**

(12) **EUROPEAN PATENT APPLICATION**

(43) Date of publication:
**11.03.2009 Bulletin 2009/11**

(51) Int Cl.:
**A61B 8/08** (2006.01)   **G01S 15/89** (2006.01)
**G01S 7/539** (2006.01)

(21) Application number: **08015402.4**

(22) Date of filing: **01.09.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **06.09.2007   JP 2007231114**

(71) Applicant: **ALOKA CO., LTD.**
**Mitaka-shi**
**Tokyo 181-8622 (JP)**

(72) Inventor: **Miyasaka, Koichi**
**Mitaka-shi**
**Tokyo 181-8622 (JP)**

(74) Representative: **Heim, Hans-Karl et al**
**Weber & Heim**
**Patentanwälte**
**Irmgardstrasse 3**
**81479 München (DE)**

(54) **Ultrasound diagnosis apparatus**

(57)    A transmission and reception unit (14) controls probes (11, 12) to form an ultrasound beam (40) which intersects a bone (52) and obtains a reception signal. A beam evaluating unit (24) evaluates an intersection state of the ultrasound beam (40) with respect to the bone (52) based on a shape of an envelope of the reception signal. More specifically, the beam evaluating unit (24) determines a quality of the reception signal based on a height and a slope of a hill-like portion, included in the envelope, corresponding to the bone (52) and evaluates the intersection state of the ultrasound beam (40).

FIG. 1

**Description**

(Background)

(Technical Field)

The present invention relates to an ultrasound diagnosis apparatus for diagnosing a bone.

(Related Art)

Simple quantitative measurement of mechanical characteristics such as bone strength is desired for diagnosing bone metabolic diseases such as osteoporosis, for judging fracture risk, and for quantitatively diagnosing bone union after treatment of bone fracture.

The evaluation of bone formation and bone union depends largely on X-ray photography, but quantitatively diagnosing bone strength by means of X-ray photography is very difficult. As a method of measuring bone strength in the related art, there is known a strength test of a sample bone of a measurement target. However, this method requires an extraction operation for obtaining a sample bone, and the method is thus invasive. A method of measuring an amount of bone and a bone density has employed devices such as general-purpose X-ray, CT and DXA (dual-energy X-ray absorptiometry). However, these devices are merely means for measuring the amount of bone and cannot provide an evaluation of bone strength. Moreover, in light of the fact that tissue is irradiated with X-rays in these methods, these methods cannot be considered non-invasive.

Other attempts to quantitatively evaluate bone strength include a strain gauge method in which a strain gauge is mounted on an external fixator and the strain of the external fixator is measured; a vibration wave method in which a vibration is applied to a bone from the outside and a characteristic frequency is evaluated; and an acoustic emission method in which acoustic waves generated by a bone which has reached yield stress are detected. These methods, however, suffer from various problems in that a limitation is imposed on the treatment to which these methods can be applied, that the bone is subjected to invasion, and that evaluation precision is insufficient.

In view of the above circumstances, the inventors of the present invention have proposed an ultrasound diagnosis apparatus for noninvasively and quantitatively evaluating the mechanical characteristics of bone (refer to, for example, Japanese Patent Publication JP 2005-152079 A).

JP 2005-152079 A discloses a technique in which a plurality of ultrasound beams are formed for a bone, a plurality of echo signals corresponding to the ultrasound beams are obtained to identify a surface point corresponding to a bone surface for each echo signal, and a bent angle of bone is calculated based on the plurality of surface points obtained from the plurality of echo signals. With this configuration, this echo-tracking technique allows non-invasive and quantitative evaluation of mechanical characteristics of a bone in a living body based on shape data such as the bent angle of the bone obtained based on the echo signals.

The present inventors have researched and developed improved techniques for the echo-tracking technique of JP 2005-152079 A. In particular, the present inventors studied a technique for obtaining a satisfactory reception signal from a bone. In order to obtain a satisfactory reception signal, it is desirable to form a satisfactory ultrasound beam.

(Summary)

The present invention was conceived in view of the above-described circumstances, and an advantage of the present invention is that a technique for evaluating a formation state of an ultrasound beam with respect to a bone is provided.

According to one aspect of the present invention, there is provided an ultrasound diagnosis apparatus comprising a probe having a plurality of transducer elements which transmit and receive an ultrasound to and from a bone, a transmission and reception unit which controls the plurality of transducer elements, to form an ultrasound beam which intersects the bone, and which obtains a reception signal, and an evaluating unit which evaluates an intersection state of the ultrasound beam with respect to the bone based on a shape of an envelope of the reception signal.

According to this aspect of the present invention, an intersection state of an ultrasound beam with respect to the bone is evaluated. The intersection state includes, for example, a position of an intersection between the bone and the ultrasound beam and an angle of the ultrasound beam with respect to the bone. Based on an evaluation result of the intersection state of the ultrasound beam with respect to the bone, for example, a position and an orientation of the probe are adjusted. Alternatively, in place of or in addition to the adjustment of the position and orientation of the probe, it is also possible to electronically or mechanically adjust the position and direction of the ultrasound beam.

(Brief Description of the Drawings)

Exemplary embodiment(s) of the present invention will be described in detail based on the following figures, wherein:

FIG. 1 is a functional block diagram showing an overall structure of an ultrasound diagnosis apparatus according to a preferred embodiment of the present invention;
FIG. 2 is a diagram for explaining a diagnosis of a bone according to a preferred embodiment of the present invention;
FIG. 3 is a diagram for explaining a tomographic image corresponding to each probe;
FIG. 4 is a diagram for explaining an envelope of a reception signal;
FIG. 5 is a diagram for explaining evaluation by a beam evaluating unit; and
FIG. 6 is a diagram exemplifying a bar graph display.

(Detailed Description)

A preferred embodiment of the present invention will now be described with reference to the drawings.

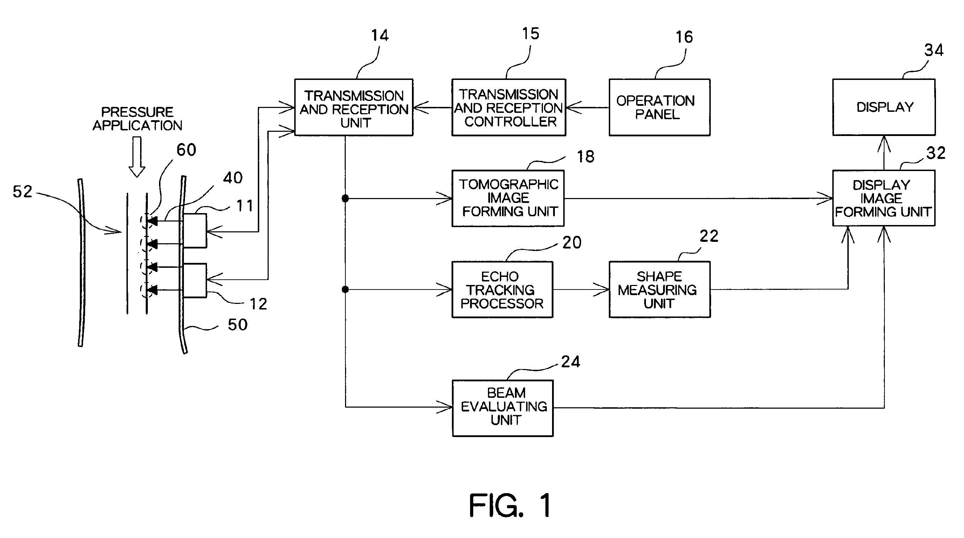

FIG. 1 shows a preferred embodiment of an ultrasound diagnosis apparatus according to the present invention. FIG. 1 is a functional block diagram showing an overall structure of the ultrasound diagnosis apparatus of the preferred embodiment of the present invention. A first probe 11 and a second probe 12 are ultrasound probes which are used in contact with a surface of a body of a subject 50. The first probe 11 and the second probe 12 are, for example, linear electronic scan probes (linear probes), and each of the first probe 11 and the second probe 12 comprises a plurality of transducer elements. The first probe 11 and the second probe 12 form ultrasound beams 40 toward a bone 52 within the body of the subject 50, electronically scan the ultrasound beams 40, and collect data for a tomographic image of the bone 52. In FIG. 1, only four ultrasound beams 40 for echo tracking to be described later are shown. For each ultrasound beam 40 for echo tracking, a surface point 60 corresponding to the surface of the bone 52 is detected.

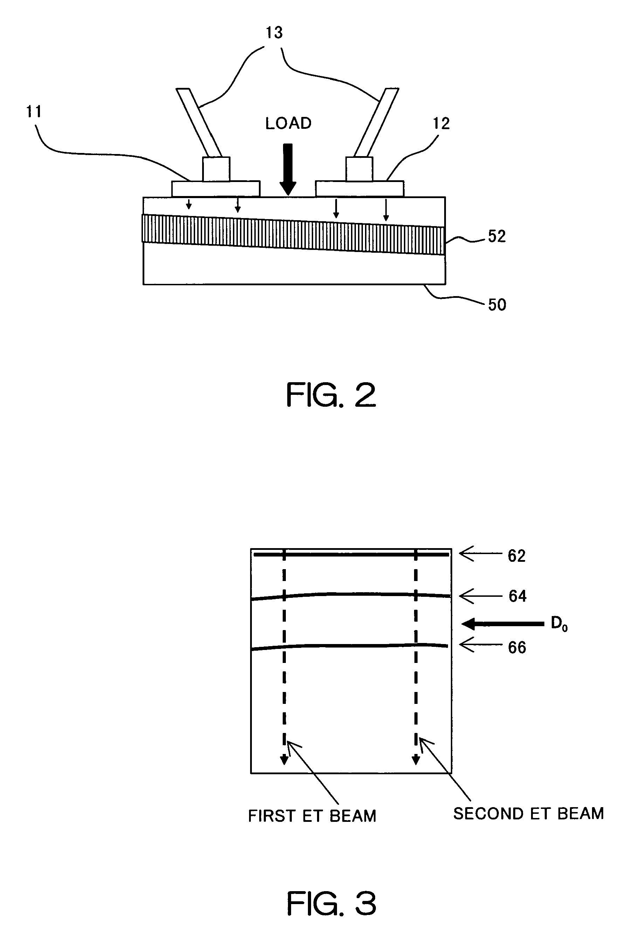

FIG. 2 is a diagram for explaining diagnosis of a bone using the ultrasound diagnosis apparatus of the present embodiment. The ultrasound diagnosis apparatus of the present embodiment is a device suitable for evaluating mechanical characteristics or the like of the bone 52 in the subject 50. The bone 52 is, for example, a fibula or a tibia within a human body.
The first probe 11 and the second probe 12 are placed in line along an axial direction of the bone 52. The first probe 11 and the second probe 12 are, for example, adhered on a surface of the body of the subject 50. An acoustic coupler or the like may be inserted between the first probe 11 and the subject 50 or between the second probe 12 and the subject 50. The first probe 11 and the second probe 12 are supported by a fixing arm 13.
During measurement, the first probe 11 and the second probe 12 are fixed to each other by the fixing arm 13 so that the first probe 11 and the second probe 12 do not move relative to each other. A load is applied from a surface of the body of the subject 50 to the bone 52 at a position between the first probe 11 and the second probe 12. Each of the first probe 11 and the second probe 12 which are placed in line along the axial direction of the bone 52 forms ultrasound beams toward the bone 52.
In this manner, in the present embodiment, mechanical characteristics or the like of the bone 52 are measured using two probes (11, 12). In some measurements, for example, inaplasticity measurement of the bone 52, only one of the first probe 11 and the second probe 12 may be used for the measurement.
Referring again to FIG. 1, the transmission and reception unit 14 controls two probes (11, 12) to electronically scan the ultrasound beams 40 in a tomographic plane (cut surface of the subject 50 shown in FIG. 1, that is, cross sectional surface along major axis of the bone 52). For example, for each probe (11, 12), a plurality of ultrasound beams 40 (only four ultrasound beams for echo tracking to be described later are shown in FIG. 1) are consecutively electronically scanned, and an echo signal (reception signal) is obtained for each ultrasound beam 40. For example, for each ultrasound beam 40, a phase-array addition process is applied to signals obtained from the plurality of transducer elements and an echo signal is formed. The plurality of echo signals obtained through the plurality of ultrasound beams 40 are output to a tomographic image forming unit 18, and the tomographic image forming unit 18 forms a tomographic image (B mode image) of the bone based on the plurality of echo signals. The formed B mode image is displayed on a display 34 through a display image forming unit 32.
The echo signal obtained in the transmission and reception unit 14 is also output to an echo tracking processor 20. The echo tracking processor 20 applies an echo tracking process in which a bone surface portion is extracted from each echo signal and is tracked. For the echo tracking process, for example, the technique detailed in JP 2001-309918 A may be used. This technique will briefly be described next.
The echo signal obtained from the probes (11, 12) has a large amplitude on a portion corresponding to a bone surface. When the bone surface portion is captured simply as a portion having a large amplitude, it is not clear which part in the area of the large amplitude corresponds to the surface portion. As a result, an extraction error in a degree of area of the large amplitude (approximately 0.2 mm in a normal ultrasound diagnosis apparatus) occurs. In the echo tracking process, a zero-cross point is detected as a representative point of the echo signal and the detected zero-cross point is tracked, so that the extraction precision is significantly improved (the precision can be improved to approximately 0.002 mm). The zero-cross point is detected as a timing in a tracking gate period in which a polarity of the amplitude of the echo signal changes from positive to negative or from negative to positive. When the zero-cross point is detected, a new tracking gate is set with the detected zero-cross point as a center. In the echo signal obtained at the next timing, the zero-cross point is detected in the newly set tracking gate period. In this manner, the zero-cross point of the echo signal is tracked as a surface point 60 for each ultrasound beam, and the position of the bone surface is highly precisely measured with the probes (11, 12) as a reference.

In the echo tracking process, for example, four tracking echo signals obtained from four ultrasound beams 40 for echo tracking are used. The position or the like of the four echo tracking ultrasound beams 40 is set, for example, by a user (inspector) controlling a transmission and reception controller 15 through an operation panel 16. The tracking echo signal may be selected from among the echo signals used for formation of the tomographic image or the tomographic image formation may be interrupted and only the four tracking echo signals may be obtained.

A shape measuring unit 22 calculates a measured amount reflecting a shape of the bone surface based on the plurality of detected surface points (tracking points) 60. An example of the measured amount would be a bent angle of the bone 52. When the bent angle or the like is to be measured, a suitable load is applied to the bone 52 (refer to FIG. 2). The load applied to the bone 52 is applied, for example, in an approximately perpendicular direction with respect to the axis of the bone 52 at a position between two probes (11, 12) while the ends of the bone 52 in the axial direction are supported. In other words, a method such as a three-point bending method in which both ends are supported and a load is applied to a point near the center is employed. The amount of load or the like should be set with great care according to the state of the bone 52.

The display image forming unit 32 forms a display image which shows the tomographic image formed by the tomographic image forming unit 18, the measured amount calculated by the shape measuring unit 22, an evaluation result by the beam evaluating unit 24, etc. The formed display image is displayed on the display 34.

The beam evaluating unit 24 evaluates a state of the ultrasound beam 40. In particular, in the present embodiment, four ultrasound beams 40 for echo tracking are evaluated. The beam evaluating unit 24 evaluates, for each echo tracking ultrasound beam 40, an intersection state of the ultrasound beam 40 with respect to the bone 52. The evaluation of the intersection state of the ultrasound beam 40 in the present embodiment will now be described in detail. For elements (structures) already shown in FIG. 1, reference numerals identical to those in FIG. 1 will be used.

As shown in FIG. 2, the first probe 11 and the second probe 12 are placed in line along the axial direction of the bone 52, and tomographic images corresponding to the first probe 11 and the second probe 12 are formed.

FIG. 3 is a diagram for explaining a tomographic image corresponding to each probe. FIG. 3 shows a tomographic image corresponding to one of the two probes (11, 12), for example, the first probe 11. The tomographic image is formed by the tomographic image forming unit 18 based on an echo signal obtained by the first probe 11 electronically and linearly scanning the ultrasound beam 40, and is displayed on the display 34 through the display image forming unit 32.

The tomographic image of FIG. 3 shows a probe surface 62, a skin surface 64 of the subject 50, and a bone surface 66 in the subject 50. The tomographic image of FIG. 3 also shows two echo tracking ultrasound beams formed by the first probe 11, that is, a first ET beam and a second ET beam. FIG. 3 shows a tomographic image when an acoustic coupler (not shown) is provided between the probe surface 62 and the subject 50.

A user (inspector) who uses the ultrasound diagnosis apparatus of the present embodiment moves a pointer of a mouse or the like on, for example, an image while viewing the tomographic image of FIG. 3 displayed on the display 34, and sets a depth Do between the skin surface 64 and the bone surface 66. Alternatively, it is also possible to place a scroll bar in the depth direction and set the depth Do through the scroll bar.

When the depth Do is set, the user switches the ultrasound diagnosis apparatus to a transmission mode for echo tracking using the operation panel 16 or the like and sets the ultrasound beams for echo tracking (first ET beam and second ET beam). A reception signal is then obtained for each ultrasound beam for echo tracking. The intersection state of each ultrasound beam is evaluated based on a shape of an envelope of the reception signal thus obtained.

Alternatively, it is also possible to employ a configuration in which the information of the depth Do which is set by the user is sent to the echo tracking processor 20 and the echo tracking processor 20 sets the position of the tracking gate which is used for the tracking process based on the depth Do.

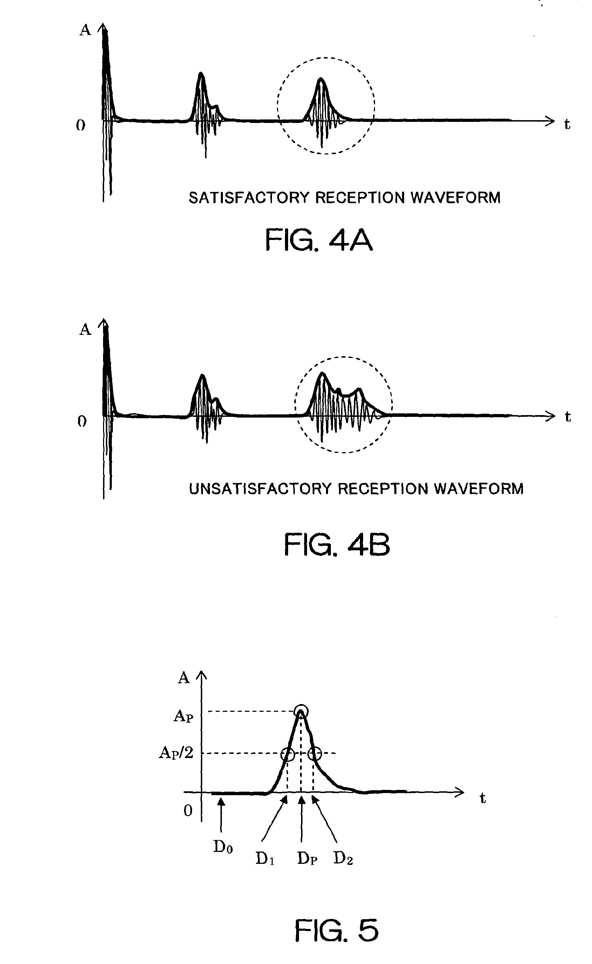

FIG. 4 is a diagram for explaining an envelope of a reception signal obtained from each ultrasound beam for echo tracking. FIG. 4A shows a reception waveform when the intersection relationship between the ultrasound beam and the bone is satisfactory. More specifically, FIG. 4A shows a receptions signal shown with a narrow line and an envelope with a wide line which is an envelope of the reception signal. FIG. 4B shows a reception waveform when the intersection relationship between the ultrasound beam and the bone is unsatisfactory. More specifically, FIG. 4B shows a reception signal shown with a narrow line and an envelope with a wide line which is an envelope of the reception signal.

Each of the envelopes of the two reception waveforms of FIGs. 4A and 4B has three hills, including a hill corresponding to a probe surface around the origin, a hill corresponding to a skin surface at a position progressed along the time axis direction from the first hill, and a hill corresponding to a bone surface which is shown surrounded by a circle of a dotted line.

In the present embodiment, an intersection relationship between the ultrasound beam and the bone is evaluated based on a shape of the hill corresponding to the bone surface in the reception waveform. In general, when the ultrasound beam intersects the bone surface in a perpendicular angle, the peak of the hill corresponding to the bone surface is large, the shape of the hill is approximately symmetric in the horizontal direction, and the slope of the hill is steep. The reception waveform shown in FIG. 4A corresponds to a waveform when the ultrasound beam is close to perpendicular to the surface of the bone.

On the other hand, for example, as the angle of the ultrasound beam with respect to the surface of the bone is deviated from a perpendicular angle, the peak of the hill corresponding to the bone surface is reduced, the shape of the hill becomes asymmetric in the horizontal direction, and the slope of the hill becomes gentle. Thereceptionwaveform- mofFIG. 4B corresponds to a waveform when the angle of the ultrasound beam with respect to the surface of the bone is significantly deviated from a perpendicular angle. The beam evaluating unit 24 evaluates the quality of the reception signal, that is, the quality of the intersection relationship between the ultrasound beam and the bone based on the shape of the hill corresponding to the bone surface included in the reception waveform shown in FIG. 4.

FIG. 5 is a diagram for explaining evaluation by the beam evaluating unit 24. FIG. 5 shows a hill corresponding to the bone surface included in the reception waveform and an envelope near the hill (corresponding to the portion surrounded by a circle of a dotted line in FIG. 4). The beam evaluating unit 24 searches, on the time axis shown in FIG. 5, for a first peak value $A_P$ along a direction toward a deeper depth, that is, a positive direction on the time axis from a position of the depth Do (refer to FIG. 3) which is set by the user, and records the position (depth) $D_P$ of the peak value $A_P$ on the time axis. The depth $D_P$ is assumed to be the peak position of the hill corresponding to the bone surface.

The beam evaluating unit 24 further searches, from the depth $D_P$ toward a shallower depth, for a depth in which the amplitude is, for example, $A_P/2$ and sets the found depth as $D_1$. The beam evaluating unit 24 then searches, from the depth $D_P$ toward a deeper depth, for a depth in which the amplitude is, for example, $A_P/2$, and sets the found depth as $D_2$. The beam evaluating unit 24 calculates a slope $\Delta_1$ of the left-side inclination of the hill and the slope $\Delta_2$ of the right-side inclination of the hill with the following equations.

$$\Delta_1 \;=\; |0.5 * A_P / (D_P - D_1)| \qquad (1)$$

$$\Delta_2 \;=\; |0.5 * A_P / (D_2 - D_P)| \qquad (2)$$

The beam evaluating unit 24 determines that the reception signal from the bone surface is satisfactory when the peak value $A_P$ and the slopes $\Delta_1$ and $\Delta_2$ satisfy the following equation.

$$A_{MIN} \;<\; A_P \text{ and } \Delta_{1MIN} \;<\; \Delta_1 \text{ and } \Delta_{2MIN} \;<\; \Delta_2 \qquad (3)$$

The values $A_{MIN}$, $\Delta_{1MIN}$, and $\Delta_{2MIN}$ which form the determination standard are suitably set according to, for example, the type of the bone to be diagnosed and the diagnosis content. Alternatively, it is also possible to employ a configuration in which the user can suitably correct these determination standards.

The beam evaluating unit 24 determines the quality of the reception signal of each ultrasound beam, based on the equations (1) - (3), for example, for all of four ultrasound beams for echo tracking. When the beam evaluating unit 24 determines that the reception signal is satisfactory, for example, the beam evaluating unit 24 displays, on the display 34, through the display image forming unit 32, that the reception signal is satisfactory. Alternatively, it is also possible to light a lamp or the like on the operation panel 16 indicating that the reception signal is satisfactory. Alternatively, it is also possible to employ a configuration in which the lamp or the like is caused to blink until the beam evaluating unit 24 determines that the reception signal is satisfactory and the lamp may be switched from blinking to continuous lighting when the beam evaluating unit 24 determines that the reception signal is satisfactory.

When the reception signal is not satisfactory, the user adjusts, for example, the position and the orientation of the probe, to adjust the intersection state between the ultrasound beam and the bone. In place of or in addition to the adjustment of the position and orientation of the probe, it is also possible to electronically or mechanically adjust the position and direction of the ultrasound beam.

It is also possible to employ a configuration in which a bar graph or a meter is displayed on the display 34 or the like indicating the degree of quality of the reception signal, so that the user can visually understand whether the adjustment of the probe or the like by the user is in a direction improving or degrading the quality of the reception signal.

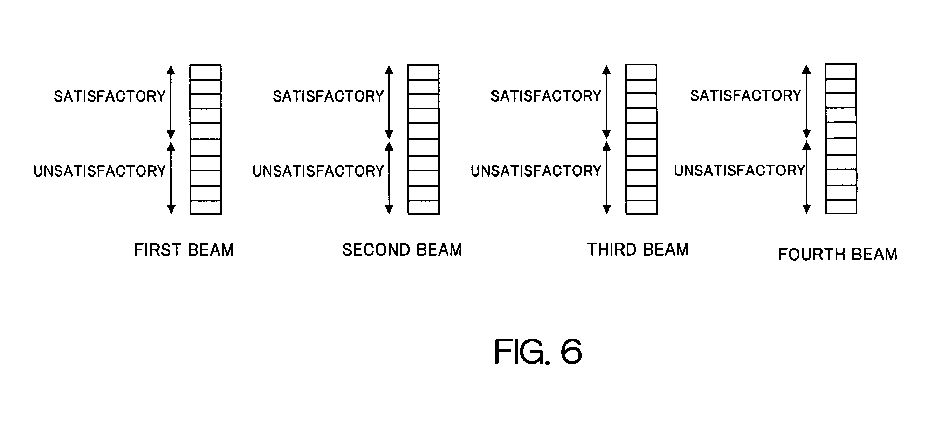

FIG. 6 is a diagram exemplifying a bar graph display. When the bar graph is to be formed, the beam evaluating unit 24 converts, for example, the values of the peak value $A_P$ and the slopes $\Delta_1$ and $\Delta_2$ into points. For example, points such as 40 points, 30 points, and 30 points may be distributed to the three information of $A_P$, $\Delta_1$, and $\Delta_2$. The total point is set, for example, at 100 points.

The numerical measurement range of the peak value $A_P$ is set to 0% to 100%, and 40 points are set to $A_P$ when a value of 100% is obtained. The maximum values for $\Delta_1$ and $\Delta_2$ are, in angles, +90° and -90°, respectively. Therefore, the angles 0° ~ +90° of $\Delta_1$ are correlated to 0% to 100%, the angles 0° ~ -90° of $\Delta_2$ are correlated to 0% to 100%, and, for example, when a value of 100% is obtained, 30 points are set to $\Delta_1$ or $\Delta_2$. A total of the obtained points of the three information of $A_P$, $\Delta_1$, and $\Delta_2$ is displayed as a bar graph. The points are calculated for each ultrasound beam.

FIG. 6 shows bar graphs corresponding to the four echo tracking ultrasound beams (first beam - fourth beam). For example, the height of the bar graph corresponding to the beam (number of lighted display segments) is changed based on the points for the beam. With this structure, the user can understand, based on the height of the bar graph, whether or not the reception signal is satisfactory for each beam or whether or not the adjustment is in a direction of improving the reception signal.

A preferred embodiment of the present invention has been described. With the preferred embodiment of the present invention, it is possible to adjust, for example, the position of the probe or the like so that the reception signal is improved, without depending on the experience or the like of the user. In addition, because the quality determination is automated, the user does not need to determine the quality of the waveform by viewing the shape of the actual waveform or the like, and, thus, the load of the user is reduced. Moreover, the time required for adjustment of the position or the like of the probe can be shortened and the measurement time is shortened, and, as a consequence, the load to the subject is also reduced.

The above-described embodiment is merely exemplary in all aspects, and is not intended to limit the scope of the present invention. In the present invention, for example, it is also possible to employ a configuration in which a portion corresponding to the surface of the bone included in the envelope of a satisfactory reception signal is set as a reference waveform, a correlation value between a portion corresponding to the surface of the bone included in the envelope of the actually obtained reception signal and the reference waveform is calculated, and the quality of the reception signal is determined based on the correlation value.

Alternatively, the determination result of the equality may be output as a sound. For example, four types of sound intervals may be prepared corresponding to the four ultrasound beams for echo tracking, and sound corresponding to the ultrasound beam may be muted when the reception signal of the ultrasound beam is satisfactory. The present invention includes various modifications that falls in the scope and spirit of the present invention.

**Claims**

1. An ultrasound diagnosis apparatus comprising:

   a probe having a plurality of transducer elements which transmit and receive an ultrasound to and from a bone;
   a transmission and reception unit which controls the plurality of transducer elements, to form an ultrasound beam which intersects the bone, and which obtains a reception signal; and
   an evaluating unit which evaluates an intersection state of the ultrasound beam with respect to the bone based on a shape of an envelope of the reception signal.

2. The ultrasound diagnosis apparatus according to Claim 1, wherein
   the evaluating unit evaluates the intersection state based on a shape of a portion, included in the envelope, corresponding to a surface of the bone.

3. The ultrasound diagnosis apparatus according to Claim 2, wherein
   the evaluating unit evaluates the intersection state based on a height of a hill-like portion, included in the envelope, corresponding to the surface of the bone.

4. The ultrasound diagnosis apparatus according to Claim 2, wherein
   the evaluating unit evaluates the intersection state based on a slope of a hill-like portion, included in the envelope, corresponding to the surface of the bone.

**5.** The ultrasound diagnosis apparatus according to Claim 2, wherein
the evaluating unit evaluates the intersection state based on a height and a slope of a hill-like portion, included in the envelope, corresponding to the surface of the bone.

**6.** The ultrasound diagnosis apparatus according to Claim 5, wherein
the evaluating unit determines that the reception signal from the surface of the bone is satisfactory and the intersection state is satisfactory when the height and the slope of the hill-like portion are superior compared to respective determination standards.

**7.** The ultrasound diagnosis apparatus according to Claim 5, wherein
the evaluating unit calculates an evaluation value related to the intersection state based on the height and the slope of the hill-like portion, and
the evaluation value calculated by the evaluating unit is displayed in a form of a graph.

**8.** The ultrasound diagnosis apparatus according to Claim 1, wherein
the transmission and reception unit forms a plurality of ultrasound beams which intersect the bone and obtains a reception signal for each of the ultrasound beams,
the ultrasound diagnosis apparatus further comprises a surface tracking unit which detects a surface point corresponding to a surface of the bone based on a reception signal obtained for each of the ultrasound beams and tracks a plurality of the surface points detected based on a plurality of the ultrasound beams, and
the evaluating unit evaluates the intersection state with respect to the bone based on a shape of an envelope of the reception signal for each ultrasound beam of the plurality of the ultrasound beams which are used for tracking the plurality of surface points.

**9.** The ultrasound diagnosis apparatus according to Claim 8, wherein
the evaluating unit evaluates the intersection state based on a shape of a portion, included in the envelope, corresponding to the surface of the bone for each of the ultrasound beams.

**10.** The ultrasound diagnosis apparatus according to Claim 9, wherein
the evaluating unit evaluates the intersection state based on a height and a slope of a hill-like portion, included in the envelope, corresponding to the surface of the bone, for each of the ultrasound beams.

**11.** The ultrasound diagnosis apparatus according to Claim 10, wherein
the evaluating unit determines that the reception signal from the surface of the bone is satisfactory and the intersection state is satisfactory when the height and the slope of the hill-like portion are superior compared to respective determination standards, for each of the ultrasound beams.

**12.** The ultrasound diagnosis apparatus according to Claim 10, wherein
the evaluating unit calculates an evaluation value related to the intersection state based on the height and the slope of 19 the hill-like portion for each of the ultrasound beams, and
the evaluation value calculated by the evaluating unit is displayed in a form of a graph for each of the ultrasound beams.

FIG. 1

## FIG. 2

LOAD

13

11

12

52

50

## FIG. 3

62

64

$D_0$

66

FIRST ET BEAM

SECOND ET BEAM

SATISFACTORY RECEPTION WAVEFORM

FIG. 4A

UNSATISFACTORY RECEPTION WAVEFORM

FIG. 4B

FIG. 5

FIG. 6

Europäisches
Patentamt

European
Patent Office

Office européen
des brevets

## EUROPEAN SEARCH REPORT

Application Number

EP 08 01 5402

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/043290 A1 (GOEPP JULIUS G [US] ET AL) 22 February 2007 (2007-02-22)<br>* paragraphs [0002], [0023], [0063], [0066], [0077] - [0079], [0097], [0109] *<br>* figures 1A-1F,3,3A,5-13 *<br>----- | 1-12 | INV.<br>A61B8/08<br>G01S15/89<br><br>ADD.<br>G01S7/539 |
| A | EP 0 873 717 A (IGEA SRL [IT]) 28 October 1998 (1998-10-28)<br>* abstract *<br>* column 7, line 46 - column 8, line 35 *<br>* claim 6 *<br>* figures 6a,6b *<br>----- | 1-12 | |
| D,A | JP 2005 152079 A (ALOKA CO LTD) 16 June 2005 (2005-06-16)<br>* abstract *<br>----- | 1-12 | |

TECHNICAL FIELDS
SEARCHED (IPC)

A61B
G01S

The present search report has been drawn up for all claims

| Place of search | Date of completion of the search | Examiner |
|---|---|---|
| Munich | 10 November 2008 | Willig, Hendrik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

EP 2 033 579 A1

## ANNEX TO THE EUROPEAN SEARCH REPORT
## ON EUROPEAN PATENT APPLICATION NO.

EP 08 01 5402

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2008

| Patent document cited in search report | | Publication date | Patent family member(s) | | Publication date |
|---|---|---|---|---|---|
| US 2007043290 | A1 | 22-02-2007 | NONE | | |
| EP 0873717 | A | 28-10-1998 | IT | T0970362 A1 | 26-10-1998 |
| | | | US | 6436042 B1 | 20-08-2002 |
| JP 2005152079 | A | 16-06-2005 | JP | 4153407 B2 | 24-09-2008 |

EPO FORM P0459

For more details about this annex : see Official Journal of the European Patent Office, No. 12/82

13

**REFERENCES CITED IN THE DESCRIPTION**

**Patent documents cited in the description**

• JP 2005152079 A

• JP 2001309918 A